(19) Europäisches Patentamt
European Patent Office
Office européen des brevets

(11) Veröffentlichungsnummer: **0 182 737 A2**

# (12) EUROPÄISCHE PATENTANMELDUNG

(21) Anmeldenummer: **85810464.9**

(22) Anmeldetag: **10.10.85**

(51) Int. Cl.[4]: **C 07 D 207/34**, C 07 D 207/33, A 01 N 43/36, C 07 C 121/43, C 07 C 103/46, C 07 C 147/06, C 07 C 21/24, C 07 C 25/24 // C07C33/46, C07C69/63, C07C69/618, C07C121/70

(30) Priorität: **16.10.84 CH 4949/84**

(43) Veröffentlichungstag der Anmeldung: **28.05.86 Patentblatt 86/22**

(84) Benannte Vertragsstaaten: **AT BE CH DE FR GB IT LI LU NL SE**

(71) Anmelder: **CIBA-GEIGY AG, Klybeckstrasse 141, CH-4002 Basel (CH)**

(72) Erfinder: **Martin, Pierre, Dr., Meisenweg 38, CH-4310 Rheinfelden (CH)**
Erfinder: **Lang, Robert Werner, Dr., Hagenbachweg 10, CH-4133 Pratteln (CH)**

(54) **3-Phenylpyrrolderivate, Verfahren zu ihrer Herstellung und ihre Verwendung als Schädlingsbekämpfungsmittel.**

(57) Neue 3-Phenylpyrrol-Derivate der Formel I

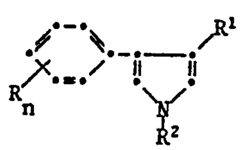

(I),

worin $R^1$ für Cyan, Trifluormethyl oder $C_1$-$C_4$-Alkoxycarbonyl, $R^2$ für Vinyl oder eine Gruppe $-CH_2-CH_2-X$, in der X Chlor, Brom, Cyan, $C_1$-$C_4$-Alkoxycarbonyl, Phenylsulfonyl, 4-Bromphenylsulfonyl oder 4-Methylphenylsulfonyl bedeutet, R für Halogen, $C_1$-$C_4$-Alkyl oder $C_1$-$C_4$-Halogenalkyl und n für eine Zahl null, eins oder zwei stehen, haben wertvolle mikrobizide Eigenschaften zur Bekämpfung von phytopathogenen Pflanzenschädlingen, insbesondere von Phytopathogenen Pilzen.

EP 0 182 737 A2

CIBA-GEIGY AG
Basel (Schweiz)

5-15112/=/ZFO

## Schädlingsbekämpfungsmittel

Die vorliegende Erfindung betrifft neue 3-Phenylpyrrol-Derivate, sie als Wirkstoff enthaltende Mittel zur Bekämpfung von phytopathogenen Mikroorganismen, die Verwendung dieser Wirkstoffe, sowie Verfahren und Zwischenprodukte zur Herstellung der neuen Verbindungen.

Die erfindungsgemässen 3-Phenylpyrrol-Derivate entsprechen der Formel I

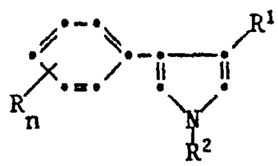

(I) ,

worin $R^1$ für Cyan, Trifluormethyl oder $C_1$-$C_4$-Alkoxycarbonyl, $R^2$ für Vinyl oder eine Gruppe $-CH_2-CH_2-X$, in der X Chlor, Brom, Cyan, $C_1$-$C_4$-Alkoxycarbonyl, Phenylsulfonyl, 4-Bromphenylsulfonyl oder 4-Methylphenylsulfonyl bedeutet, R für Halogen, $C_1$-$C_4$-Alkyl oder $C_1$-$C_4$-Halogenalkyl und n für eine Zahl null, eins oder zwei steht.

N-substituierte Pyrrolverbindungen sind in der Literatur bereits für verschiedene Verwendungszwecke beschrieben worden. Derartige Verbindungen sind als Stabilisatoren für Kunststoffe aus der GB-PS 2.078.761 oder als Polymerisationskatalysatoren aus der DE-OS 2.028.363 bekannt. Kürzlich sind N-sulfenylierte 3-Phenylpyrrol-Verbindungen beispielsweise in der publizierten Europäischen Patentanmeldung EP-A-96 142 als Mikrobizide vorgestellt worden.

Im Rahmen der vorliegenden Erfindung steht der Begriff Alkoxycarbonyl für Methoxycarbonyl, Aethoxycarbonyl, i-Propyloxycarbonyl, n-Propyloxycarbonyl sowie die vier isomeren Butyloxycarbonyl. Bevorzugt sind Methoxy- oder Aethoxycarbonyl. Die gleiche Definition und Bevorzugung gilt für den Begriff Alkoxycarbonyläthyl, der unter dem Symbol $R^2$ konstruierbar ist. Hier sind die bevorzugten Formen Methoxycarbonyläthyl oder Aethoxycarbonyläthyl. Halogen steht für Fluor, Chlor, Brom oder Jod, vorzugsweise aber für Chlor. Unter Alkyl ist zu verstehen: Methyl, Aethyl, i-Propyl, n-Propyl sowie die vier isomeren Butyl. Beispiele für Halogenalkyl sind: Fluormethyl, Difluormethyl, Trifluormethyl, Chlormethyl, Trichlormethyl, 2,2,2-Trifluoräthyl, 3,3,3-Trichloräthyl, 2-Chloräthyl, Perfluoräthyl, 2-Fluoräthyl oder 1,1-Dichlor-2,2,2-trifluoräthyl.

Von den erfindungsgemässen Verbindungen der Formel I sind diejenigen bevorzugt, in denen entweder

a) die Phenylgruppe in 2- und 3-Stellung durch zwei Chloratome substituiert ist, oder

b) $R^1$ für Cyan oder Trifluormethyl steht, oder

c) $R^2$ für Aethoxycarbonyläthyl oder 2-Cyanoäthyl

steht.

Ganz besonders bevorzugt ist eine Gruppe von Wirkstoffen der Formel I, in denen die Phenylgruppe in 2- und 3-Stellung durch Chloratome substituiert ist, $R^1$ für Trifluormethyl oder Cyan und $R^2$ für Aethoxycarbonyläthyl oder 2-Cyanoäthyl stehen.

Als bevorzugte Einzelverbindungen sind zu nennen:

1-Aethoxycarbonyläthyl-3-(2,3-dichlorphenyl)-4-trifluormethyl-pyrrol oder

1-Aethoxycarbonyläthyl-4-cyano-3-(2,3-dichlorphenyl)-pyrrol.

Die neuen Verbindungen der Formel I werden erfindungsgemäss hergestellt, indem man ein Formylglycin-Derivat der Formel II

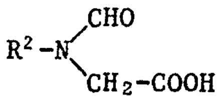

(II) ,

worin $R^2$ die unter Formel I gegebene Bedeutung hat, in Gegenwart eines Kondensationsmittels mit einem Phenylacetylen-Derivat der Formel III

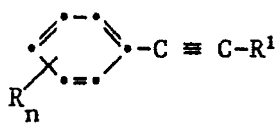

(III) ,

worin $R^1$, R und n die unter Formel I gegebenen Bedeutungen haben, behandelt.

Als Kondensationsmittel eignet sich in besonderer Weise Essigsäureanhydrid. Es können aber auch Reagenzien wie Dicyclohexylcarbodiimid, Diisopropylcarbodiimid, Trifluoressigsäureanhydrid oder Propionsäureanhydrid eingesetzt werden. Das Verfahren wird dabei entweder in einem inerten organischen Lösungsmittel wie Toluol, Xylol oder Mesitylen, oder wahlweise ohne Lösungsmittel in einem Ueberschuss des Kondensationsmittels durchgeführt. Die Reaktionstemperaturen liegen dabei im allgemeinen bei der Siedetemperatur des Reaktionsgemisches. Je nach Lösungsmittel wird die Reaktion bei einer Temperatur zwischen 90° und 150°C, vorzugsweise zwischen 110° und 140+C, durchgeführt.

Die Formylglycin-Derivate der Formel II sind neu und wurden speziell für die Synthese der Verbindungen der Formel I entwickelt und hergestellt. Sie bilden daher ebenfalls einen Gegenstand der vorliegenden Erfindung.

Man erhält diese Verbindungen in an sich bekannter Weise mit Vorteil durch Formylierung eines Glycinesters der Formel

$R^2$-NH-$CH_2$-COOT ,

worin $R^2$ die unter Formel I gegebene Bedeutung hat und T für $C_1$-$C_4$-Alkyl steht, mit Ameisensäure oder durch Formylierung des entsprechenden Glycinhydrochlorids mit einem Alkaliformiat und anschliessende Verseifung der Estergruppe.

Die Verbindungen der Formel III, in denen $R^1$ für Cyan oder $C_1$-$C_4$-Alkoxycarbonyl steht, sind bekannt oder können analog zu bekannten Verfahren durch Bromaddition und anschliessende doppelte Eliminierung von Bromwasserstoff aus den entsprechenden Derivaten der Zimtsäure erhalten werden.

Neu sind dagegen diejenigen Verbindungen der Formel III, in denen $R^1$ für Trifluormethyl und n für die Zahl eins oder zwei steht. Die Synthese dieser Verbindungen wurde speziell für die Herstellung der Verbindungen der Formel I entwickelt. Die neuen Verbindungen der Formel IIIa

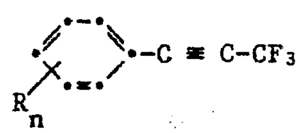 (IIIa) ,

worin $R^1$ für Trifluormethyl und n für die Zahl eins oder zwei steht, sowie das Herstellungsverfahren unter Verwendung dieser Zwischenprodukte und weitere neue Zwischenprodukte bilden daher ebenfalls Aspekte der vorliegenden Erfindung.

Man erhält nach einem erfindungsgemässen Verfahren die 3-Phenylpyrrol-Derivate der Formel I, worin $R^1$ für Trifluormethyl steht, indem man aus einem Styrolderivat der Formel IV

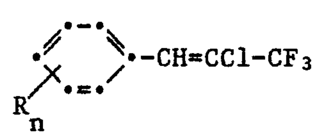 (IV) ,

worin R und n die unter Formel I gegebene Bedeutung haben, in Gegenwart einer Base Chlorwasserstoff abspaltet und das entstandene Trifluormethyl-phenylacetylen-Derivat der Formel IIIa

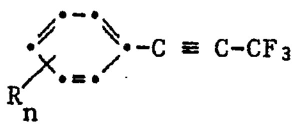 (IIIa) ,

worin R und n die unter Formel I gegebene Bedeutung haben, in Gegenwart eines Kondensationsmittels mit einem Formylglycin-Derivat der Formel II

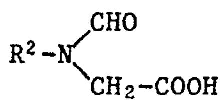 (II) ,

worin $R^2$ die unter Formel I gegebene Bedeutung hat, umsetzt.

Die Reaktionsbedingungen im ersten Schritt des obigen Verfahrens (IV ⟶ IIIa, Chlorwasserstoffabspaltung) entsprechen der üblichen Technik: so kann man eine grosse Anzahl von Basen wie anorganische Alkali- und Erdalkalihydroxide, -oxide, -hydrogencarbonate oder -carbonate oder organische Basen wie tertiäre Amine, einsetzen, sowie in einem inerten polaren, protischen oder aprotischen Lösungsmittel arbeiten. Einige typische Reaktionsbedingungen für Eliminierungsreaktionen dieses Typs sind durch folgende Reagenzienkombinationen gekennzeichnet: z.B. Kaliumhydroxid in Methanol, Aethanol oder Isopropanol; Natriummethylat in Methanol; Kalium-tert.butylat in tert.Butanol; Kalium-tert.butylat in Aethanol; Natriumamid in flüssigem Ammoniak; Kaliumhydroxid in Aethylenglykoldimethyläther; Natriumhydroxid in Wasser; oder Natriumhydroxid in Dimethylsulfoxid. Mit Vorteil werden die Reaktionsgemische zur Durchführung der Eliminierungsreaktionen erwärmt bzw. zum Rückfluss erhitzt. Der zweite Reaktionsschritt wird gemäss den bereits weiter oben beschriebenen Reaktionsbedingungen für die Umsetzung (II + III ⟶ I) durchgeführt.

Die Ausgangsprodukte der Formel IV sind ebenfalls neu. Man kann sie beispielsweise durch Reaktionen gemäss dem folgenden Reaktionsschema 1 erhalten:

Schema 1:

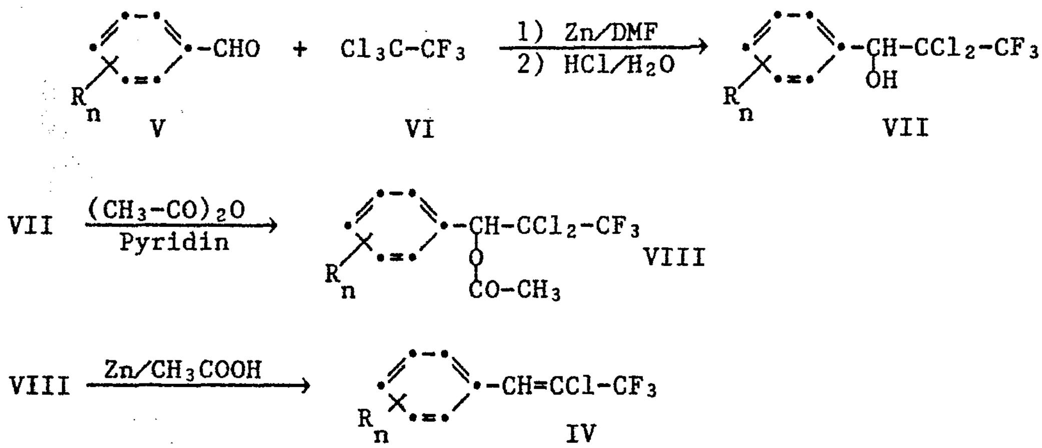


V VI VII

VIII

IV

Die gemäss Schema 1 durchlaufenen Zwischenprodukte der Formeln VII und VIII, worin R und n die unter Formel I gegebenen Bedeutungen haben, sind in der Literatur noch nicht beschrieben. Sie bilden gleichfalls einen Gegenstand der Erfindung.

Die erfindungsgemässen, neuen Pyrrolderivate der Formel I stellen eine wertvolle Bereicherung des Standes der Technik dar, denn es wurde überraschend festgestellt, dass die neuen Pyrrole der Formel I ein für Freiland-Bedürfnisse sehr günstiges Mikrobizid-Spektrum gegen phytopathogene Pilze und Bakterien aufweisen. Sie können aber nicht nur im Ackerbau oder ähnlichen Einsatzgebieten zur Bekämpfung schädlicher Mikroorganismen an Kulturpflanzen, sondern zusätzlich im Vorratsschutz zur Konservierung verderblicher Waren eingesetzt werden. Verbindungen der Formel I besitzen sehr wertvolle kurative, systemische und insbesondere präventive Eigenschaften und lassen sich zum Schutz von zahlreichen, insbesondere Freilandkulturen, einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind beispielsweise gegen die den folgenden Klassen angehörenden phytopathogenen Pilze wirksam: Ascomytes z.B. Erysiphe, Sclerotinia, Fusarium, Monilinia, Helminthosporium; Basidiomycetes wie z.B. Puccinia, Tilletia, Rhizoctonia; sowie die der Klasse der Phycomycetes angehörenden Oomycetes wie Phytophthora. Als Pflanzenschutzmittel können die Verbindungen der Formel I mit besonders gutem Erfolg gegen wichtige Schadpilze aus der Familie der Fungi imperfecti eingesetzt werden, so z.B. gegen Cercospora, Piricularia und vor allem gegen Botrytis. Botrytis spp. (B. cinerea, B. allii) stellt mit der Grauschimmelfäule an Reben, Erdbeeren, Aepfeln, Zwiebeln und anderen Obst- und Gemüsesorten einen bedeutenden wirtschaftlichen Schadfaktor dar. Darüberhinaus lassen sich einige Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida.

Als Pflanzenschutzmittel besitzen die Verbindungen der Formel I ein für die praktische Anwendung in der Landwirtschaft sehr günstiges Wirkungsspektrum zum Schutze von Kulturpflanzen, ohne diese durch unerwünschte Nebenwirkungen nachteilig zu beeinflussen.

Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Darüberhinaus schliesst die vorliegende Erfindung auch die Herstellung (agro)chemischer Mittel ein, die gekennzeichnet ist durch das innige Vermischen der Aktivsubstanz mit einem oder mehreren hierin beschriebenen Substanzen bzw. Substanzgruppen von Träger- oder Zuschlagstoffen. Eingeschlossen ist auch ein Verfahren zur Behandlung von Pflanzen oder Vorratsgütern, das sich durch Applikation der Verbindungen der Formel I bzw. der neuen Mittel auf die Pflanzen, Pflanzenteile, deren Standort oder das Substrat kennzeichnet.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren): Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusem, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Als Vorratsschutzmittel werden die Verbindungen der Formel I in unveränderter Form oder vorzugsweise zusammen mit den in der Formulierungstechnik üblichen Hilfsmitteln eingesetzt und werden z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen und die Form des Mittels werden den angestrebten Zielen und den gegebenen Verhältnissen angepasst. Günstige Aufwandmengen liegen im allgemeinen bei 0,01 bis höchstens 2 kg Aktivsubstanz pro 100 kg zu schützendes Substrat; sie

hängen jedoch ganz wesentlich von der Beschaffenheit (Grösse der Oberfläche, Konsistenz, Feuchtigkeitsgehalt) des Substrats und dessen Umgebungseinflüssen ab.

Unter Lager- und Vorratsgütern sollen im Rahmen vorliegender Erfindung pflanzliche und/oder tierische Naturstoffe und deren Weiterverarbeitungsprodukte verstanden werden, beispielsweise die nachfolgend aufgezählten und aus dem natürlichen Lebenszyklus herausgenommenen Pflanzen, deren Pflanzenteile (Stengel, Blätter, Knollen, Samen, Früchte, Körner), die im frisch geernteten Zustand oder in weiterverarbeitbarer Form vorliegen (vorgetrocknet, befeuchtet, zerkleinert, gemahlen, geröstet). Als Beispiele, die keinen das Anwendungsgebiet limitierenden Charakter im Rahmen dieser Erfindung besitzen, seien folgende Ertragsgüter genannt: Getreide (wie Weizen, Gerste, Roggen, Hafer, Reis, Sorghum und Verwandte); Rüben (wie Möhren, Zucker- und Futterrüben); Kern-, Stein- und Beerenobst (wie Aepfel, Birnen, Pflaumen, Mandeln und Kirschen; Erd-, Him- und Brombeeren); Hülsenfrüchte (wie Bohnen, Linsen, Erbsen, Soja); Oelkulturen (wie Raps, Senf, Mohn, Sonnenblumen, Kokos, Rizinus, Kakao, Erdnüsse); Gurkengewächse (wie Kürbis, Gurken, Melonen); Fasergewächse (wie Baumwolle, Flachs. Hanf, Jute, Nessel); Citrusfrüchte; Gemüsesorten (wie Spinat, Salat, Spargel, Kohlarten, Zwiebeln, Tomaten, Kartoffeln, Paprika); Lorbeergewächse (wie Avocadom Cinnamonum, Kampfer) oder Mais, Tabak, Nüsse, Kaffee, Zuckerrohr, Tee, Weintrauben, Kastanien, Hopfen, Bananen. Gras und Heu.

Als Naturprodukte tierischer Herkunft seien insbesondere getrocknete Fleisch- und Fischverarbeitungsprodukte wie Trockenfleisch, Trockenfisch, Fleischkonzentrate, Knochenmehl, Fischmehl und Tiertrockenfutter genannt.

Das behandelte Vorratsgut wird durch Behandlung mit Verbindungen der Formel I nachhaltig vor dem Befall durch Schimmelpilze und andere unerwünschte Mikroorganismen geschützt. Dadurch wird die Bildung toxischer und zum Teil karzinogener Schimmelpilze bzw. ihre Stoffwechselprodukte, wie Aflatoxine und Ochratoxine, unterbunden, das Gut vor dem Verderben bewahrt und dessen Qualität für längere Zeit aufrechterhalten. Das erfindungsgemässe Verfahren kann auf alle trockenen und feuchten Vorrats- und Lagergüter angewendet werden, die gegen Mikroorganismen, wie Hefen, Bakterien und insbesondere Schimmelpilze anfällig sind.

Ein bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Konservierungs-Verfahren ist ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche, Pflanze oder Substrat gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines (agro)chemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden

Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen, in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl, Sonnenblumenöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruh, Sepiolit oder Bentonit, als nicht adsorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände, wie z.B. Korkmehl oder Sägemehl, verwendet werden.

Besonders vorteilhafte, applikationsfördernde Zuschlagstoffe, die zu einer starken Reduktion der Aufwandmenge führen können, sind fener natürliche (tierische oder pflanzliche) oder synthetische Phospholipide aus der Reihe der Kephaline und Lecithine, wie z.B. Phosphatidyläthanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere Hirn, Herz, Lunge, Leber, Eidotter oder Sojabohnen gewinnen kann. Verwendbare Handelsmischungen sind z.B. Phosphatidylcholin-Mischungen. Synthetische Phospholipide sind z.B. Dioctanoylphosphatidylcholin und Dipalmititoylphosphatidylcholin.

Als oberflächenaktive Verbindungen kommen je nach Art des zu formulierenden Wirkstoffes der Formel I nichtionogene, kation- und/oder anionaktive Tenside in guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. as Kokosnuss- oder Talgöl gewonnen werden können, genannt. Ferner sind auch die Fettsäuremethyllaurinsalz zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14-äthylenoxidadduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 g Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthylenäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid. Auf dem Vorratsschutzgebiet werden die für die menschliche und tierische Ernährung unbedenklichen Zuschlagstoffe bevorzugt.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige (agro)chemische Mitel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet:
h = Stunde; RT = Raumtemperatur; abs. = absolut, wasserfrei;
DMF = Dimethylformamid. Druckangaben erfolgen in Millibar mb, oder Bar b. THF steht für Tetrahydrofuran.

## Herstellungsbeispiele

**Beispiel H1: N-Aethoxycarbonyläthyl-N-formylglycin**

115,5 g N-Aethoxycarbonyläthyl-N-formylglycinäthylester werden in 150 ml Aethanol gelöst und auf 0° abgekühlt. Zu dieser Lösung lässt man ein Gemisch von 28,0 g Kaliumhydroxid, 60 ml Wasser und 120 ml Aethanol zutropfen. Die Reaktionsmischung wird für 18 h bei RT gerührt und anschliessend unter verminderten Druck eingedampft. Nach Zusatz einer kleinen Menge Wassers wird das Gemisch mit konzentrierter Salzsäure auf pH 1 angesäuert. Durch Extraktion mit Chloroform, Trocknen und Eindampfen der organischen Phase erhält man N-Aethoxycarbonyläthyl-N-formylglycin in Form eines gelbstichigen Oeles (Verbindung Nr. 1.3).

**Beispiel H2: N-(2-Cyanoäthyl)-N-formylglycin**

Eine Mischung von 21,0 g N-(2-Cyanoäthyl)-glycinäthylester und 18,3 g Ameisensäure wird für 2 h zum Rückfluss erhitzt. Nach Einengen dieser Mischung unter vermindertem Druck wird der Rückstand in 50 ml Aethanol aufgenommen, auf 0° abgekühlt und tropfenweise mit einer Lösung von 25,5 g Kaliumhydroxid und 40 ml Wasser in 80 ml Aethanol versetzt. Nachdem die Reaktionslösung sich bis auf RT erwärmt hat, wird das Lösungsmittel abgedampft und der Rückstand mit konz. Salzsäure bis auf pH 1 angesäuert. Nach Sättigung mit Natriumchlorid wird das Gemisch mit Aethylacetat extrahiert. Die vereinigten organischen Extrakte werden eingedampft. Man erhält so N-(2-Cyanoäthyl)-N-formylglycin, Smp. 112-114° (Verbindung Nr. 1.1).

**Beispiel H3: (2-Chlor-3,3,3-trifluorpropen-1-yl)-2,3-dichlorbenzol**

a) (2,2-Dichlor-3,3,3-trifluor-1-hydroxypropyl)-2,3-dichlorbenzol.
Eine Suspension von 35,0 g 2,3-Dichlorbenzaldehyd, 41,2 g 1,1,1-Trichlor-2,2,2-trifluoräthan und 16,4 g Zinkpulver in 360 ml DMF wird für 66 h bei RT gerührt und anschliessend in einer Mischung von 600 ml 10 %iger Salzsäure und 600 g Eis aufgenommen. Durch Extraktion mit Aether, Trocknen und Eindampfen der organischen Extrakte erhält man (2,2-Dichlor-3,3,3-trifluor-1-hydroxypropyl)-2,3-dichlorbenzol in Form eines gelben Oeles (Verbindung Nr. 5.10).

b) (1-Acetoxy-2,2-dichlor-3,3,3-trifluorpropyl)-2,3-dichlorbenzol. Eine Mischung von 57,0 g (2,2-Dichlor-3,3,3-trifluor-1-hydro-propyl)-2,3-dichlorbenzol und 39,1 g Essigsäureanhydrid wird mit 33,0 g Pyridin versetzt und für 4 h bei RT gerührt. Durch Eingiessen in Eiswasser, Ansäuern mit 10 %iger Salzsäure, Extraktion mit Aether und Trocknen und Eindampfen der ätherischen Extrakte erhält man (1-Acetoxy-2,2-dichlor-3,3,3-trifluorpropyl)-2,3-dichlorbenzol, Smp. 92-95° aus Aethanol (Verbindung Nr. 6.10).

c) Eine Lösung von 59,0 g (1-Acetoxy-2,2-dichlor-3,3,3-trifluorpro-pyl)-2,3-dichlorbenzol in 350 ml Essigsäure wird auf 40° erwärmt und portionsweise mit 13,0 g Zinkpulver versetzt. Zur vollständigen Beendigung der Reaktion wird das Reaktionsgemisch für 3 h auf 90° erhitzt. Zur Aufarbeitung wird die Reaktionsmischung in Eiswasser aufgenommen und mit Aether extrahiert. Die vereinigten organischen Phasen werden mit Wasser, Natriumhydrogencarbonatlösung und Natriumchloridlösung gewaschen, über Magnesiumsulfat getrocknet und eingedampft. Man erhält so (2-Chlor-3,3,3-trifluorpropen-1-yl)-2,3-dichlorbenzol als gelbes Oel (Verbindung Nr. 4.10).

Beispiel H4: 1-Aethoxycarbonyläthyl-3-(2,3-dichlorphenyl)-4-trifluormethylpyrrol

a) 2,3-Dichlor-(3,3,3-trifluorpropin-1-yl)-benzol. Zu einer Lösung von 31,7 g (2-Chlor-3,3,3-trifluorpropen-1-yl)-2,3-dichlorbenzol in 50 ml tert.-Butanol wird bei RT eine Lösung von 14,1 g Kalium-tert.butylat in 750 ml tert.-Butanol so zugetropft, dass die Temperatur der Reaktionslösung 30° nicht übersteigt. Nach fortgesetztem Rühren für 18 h bei RT wird das Gemisch in Eiswasser aufgenommen und mit Aether extrahiert. Die organischen Phasen werden vereinigt, mit Wasser und Natriumchlorid-Lösung gewaschen und

eingedampft. Durch Destillation im Vakuum erhält man 2,3-Dichlor-(3,3,3-trifluorpropin-1-yl)-benzol als farbloses Oel, Sdp. 92-98° bei 22 mb (Verbindung Nr. 2.27).

b) 23,9 g 2,3-Dichlor-(3,3,3-trifluorpropin-1-yl)-benzol und 20,3 g N-Aethoxycarbonyläthyl-N-formylglycin werden in 100 ml Essigsäureanhydrid gelöst und für 24 h zum Rückfluss erhitzt. Das Gemisch wird unter vermindertem Druck eingedampft, der Rückstand in Toluol aufgenommen und durch Chromatographie an Kieselgel gereinigt. Durch Abdampfen des Lösungsmittels erhält man aus dem Eluat 1-Aethoxycarbonyläthyl-3-(2,3-dichlorphenyl)-4-trifluormethylpyrrol in Form eines farblosen Oeles (Verbindung Nr. 3.117).

Beispiel H5: 4-Aethoxycarbonyl-1-(2-cyanoäthyl)-3-phenylpyrrol

17,4 g Phenylacetylencarbonsäureäthylester und 15,6 g N-(2-Cyanäthyl)-N-formylglycin werden in 100 ml Essigsäureanhydrid gelöst und für 24 h zum Rückfluss erhitzt. Das Gemisch wird unter vermindertem Druck eingedampft, der Rückstand in Toluol aufgenommen und durch Chromatographie an Kieselgel gereinigt. Durch Abdampfen des Lösungsmittels erhält man aus dem Eluat 4-Aethoxycarbonyl-1-(2-cyanoäthyl)-3-phenylpyrrol in Form eines gelben Oeles (Verbindung Nr. 3.3).

Beispiel H6: 1-(2-Cyanoäthyl)-3-(2,3-dichlorphenyl)-4-trifluormethylpyrrol

23,9 g 2,3-Dichlor-(3,3,3-trifluorpropin-1-yl)-benzol und 15,6 g N-(2-Cyanoäthyl)-N-formylglycin werden in 100 ml Essigsäureanhydrid gelöst und für 24 h zum Rückfluss erhitzt. Das Gemisch wird unter vermindertem Druck eingedampft, der Rückstand in Toluol aufgenommen und durch Chromatographie an Kieselgel gereinigt. Durch Abdampfen des Lösungsmittels erhält man aus dem Eluat 1-(2-Cyanoäthyl)-3-(2,3-dichlorphenyl)-4-trifluormethylpyrrol, Smp. 83-85° aus Aether/Hexan (Verbindung Nr. 3.28).

In analoger Weise erhält man die in den anschliessenden Tabellen aufgelisteten Zwischen- und Endprodukte.

Tabelle 1:

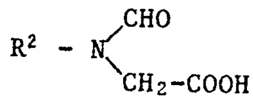

| Verb.Nr. | $R^2$ |
|---|---|
| 1.1 | $-CH_2-CH_2-CN$ |
| 1.2 | $-CH_2-CH_2-COOCH_3$ |
| 1.3 | $-CH_2-CH_2-COOC_2H_5$ |
| 1.4 | $-CH_2-CH_2-COOC_3H_7$-i |
| 1.5 | $-CH_2-CH_2-COOC_3H_7$-n |
| 1.6 | $-CH_2-CH_2-COOC_4H_9$-n |
| 1.7 | $-CH{=}CH_2$ |
| 1.8 | $-CH_2-CH_2-Cl$ |
| 1.9 | $-CH_2-CH_2-SO_2-$ 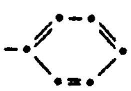 $-CH_3$ |
| 1.10 | $-CH_2-CH_2-SO_2-$ 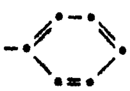 $-Br$ |
| 1.11 | $-CH_2-CH_2-SO_2-C_6H_5$ |
| 1.12 | $-CH_2-CH_2-Br$ |

Tabelle 2:

$$E - C \equiv C - R^1$$

| Verb.Nr. | E | $R^1$ |
|---|---|---|
| 2.1 | $C_6H_5-$ | $CF_3$ |
| 2.2 | $C_6H_5$ | $COOCH_3$ |
| 2.3 | $C_6H_5$ | $COOC_2H_5$ |
| 2.4 | $C_6H_5$ | CN |
| 2.5 | $2-Cl-C_6H_4-$ | $CF_3$ |
| 2.6 | $2-Cl-C_6H_4-$ | $COOC_2H_5$ |
| 2.7 | $2-Cl-C_6H_4-$ | CN |
| 2.8 | $2-Cl-C_6H_4-$ | $COOC_3H_7$-i |
| 2.9 | $3-Cl-C_6H_4-$ | $CF_3$ |
| 2.10 | $3-Cl-C_6H_4-$ | CN |
| 2.11 | $3-Cl-C_6H_4-$ | $COOC_2H_5$ |
| 2.12 | $3-Cl-C_6H_4-$ | $COOC_4H_9$-n |

Tabelle 2 (Fortsetzung)

| Verb.Nr. | E | $R^1$ |
|---|---|---|
| 2.13 | $2-CF_3-C_6H_4-$ | $COOCH_3$ |
| 2.14 | $2-CF_3-C_6H_4-$ | $COOC_2H_5$ |
| 2.15 | $2-CF_3-C_6H_4-$ | $CF_3$ |
| 2.16 | $2-CF_3-C_6H_4-$ | CN |
| 2.17 | $4-Cl-C_6H_4-$ | $COOCH_3$ |
| 2.18 | $4-Cl-C_6H_4-$ | $CF_3$ |
| 2.19 | $4-Cl-C_6H_4-$ | CN |
| 2.20 | $2-CF_3-4-Cl-C_6H_3-$ | CN |
| 2.21 | $2-CF_3-4-Cl-C_6H_3-$ | CN |
| 2.22 | $2-CF_3-4-Cl-C_6H_3-$ | $COOCH_3$ |
| 2.23 | $2-Cl-4-Cl-C_6H_3-$ | CN |
| 2.24 | $2-Cl-4-Cl-C_6H_3-$ | $CF_3$ |
| 2.25 | $2-Cl-4-Cl-C_6H_3-$ | $COOC_2H_5$ |
| 2.26 | $2-Cl-3-Cl-C_6H_3-$ | CN |
| 2.27 | $2-Cl-3-Cl-C_6H_3-$ | $CF_3$ |
| 2.28 | $2-Cl-3-Cl-C_6H_3-$ | $COOCH_3$ |
| 2.29 | $2-Cl-3-Cl-C_6H_3-$ | $COOC_2H_5$ |
| 2.30 | $2-Cl-3-Cl-C_6H_3-$ | $COOC_3H_7-i$ |
| 2.31 | $2-Cl-3-Cl-C_6H_3-$ | $COOC_3H_7-n$ |
| 2.32 | $2-Cl-3-Cl-C_6H_3-$ | $COOC_4H_9-n$ |
| 2.33 | $2-Cl-3-Cl-C_6H_3-$ | $COOC_4H_9-s$ |
| 2.34 | $2-Cl-3-Cl-C_6H_3-$ | $COOC_4H_9-i$ |
| 2.35 | $2-Cl-3-Cl-C_6H_3-$ | $CF_3$ |
| 2.36 | $2-Cl-4-CF_3-C_6H_3-$ | CN |
| 2.37 | $2-Cl-4-CF_3-C_6H_3-$ | $COOCH_3$ |
| 2.38 | $2-Cl-4-CF_3-C_6H_3-$ | $COOC_2H_5$ |
| 2.39 | $4-CH_3-C_6H_4-$ | $CF_3$ |
| 2.40 | $4-CH_3-C_6H_4-$ | CN |
| 2.41 | $4-CH_3-C_6H_4-$ | $COOCH_3$ |
| 2.42 | $2-F-C_6H_4-$ | $CF_3$ |
| 2.43 | $2-F-C_6H_4-$ | CN |
| 2.44 | $2-F-C_6H_4-$ | $COOCH_3$ |

Tabelle 3:

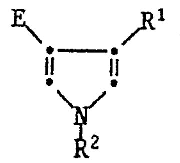

| Verb.Nr. | E | $R^1$ | $R^2$ |
|---|---|---|---|
| 3.1 | $C_6H_5-$ | $CF_3$ | $-CH_2-CH_2-CN$ |
| 3.2 | $C_6H_5-$ | $COOCH_3$ | $-CH_2-CH_2-CN$ |
| 3.3 | $C_6H_5-$ | $COOC_2H_5$ | $-CH_2-CH_2-CN$ |
| 3.4 | $C_6H_5-$ | CN | $-CH_2-CH_2-CN$ |
| 3.5 | $2-Cl-C_6H_4-$ | $CF_3$ | $-CH_2-CH_2-CN$ |
| 3.6 | $2-Cl-C_6H_4-$ | $COOC_2H_5$ | $-CH_2-CH_2-CN$ |
| 3.7 | $2-Cl-C_6H_4-$ | CN | $-CH_2-CH_2-CN$ |
| 3.8 | $2-Cl-C_6H_4-$ | $COOCH_3$ | $-CH_2-CH_2-CN$ |
| 3.9 | $2-Cl-C_6H_4-$ | $OOC_3H_7-i$ | $-CH_2-CH_2-CN$ |
| 3.10 | $3-Cl-C_6H_4-$ | $CF_3$ | $-CH_2-CH_2-CN$ |
| 3.11 | $3-Cl-C_6H_4-$ | CN | $-CH_2-CH_2-CN$ |
| 3.12 | $3-Cl-C_6H_4-$ | $COOC_2H_5$ | $-CH_2-CH_2-CN$ |
| 3.13 | $3-Cl-C_6H_4-$ | $COOC_4H_9-n$ | $-CH_2-CH_2-CN$ |
| 3.14 | $2-F.C_6H_4-$ | $CF_3$ | $-CH_2-CH_2-CN$ |
| 3.15 | $2-F-C_6H_4-$ | CN | $-CH_2-CH_2-CN$ |
| 3.16 | $2-F-C_6H_4-$ | $COOCH_3$ | $-CH_2-CH_2-CN$ |
| 3.17 | $2-CF_3-C_6H_4-$ | $COOC_2H_5$ | $-CH_2-CH_2-CN$ |
| 3.18 | $2-CF_3-C_6H_4-$ | $COOCH_3$ | $-CH_2-CH_2-CN$ |
| 3.19 | $2-CF_3-C_6H_4-$ | $CF_3$ | $-CH_2-CH_2-CN$ |
| 3.20 | $2-CF_3-C_6H_4-$ | CN | $-CH_2-CH_2-CN$ |
| 3.21 | $4-Cl-C_6H_4-$ | $COOCH_3$ | $-CH_2-CH_2-CN$ |
| 3.22 | $4-Cl-C_6H_4-$ | $CF_3$ | $-CH_2-CH_2-CN$ |
| 3.23 | $4-Cl-C_6H_4-$ | CN | $-CH_2-CH_2-CN$ |
| 3.24 | $2-Cl-4-Cl-C_6H_3-$ | $CF_3$ | $-CH_2-CH_2-CN$ |
| 3.25 | $2-Cl-4-Cl-C_6H_3-$ | CN | $-CH_2-CH_2-CN$ |
| 3.26 | $2-Cl-4-Cl-C_6H_3-$ | $COOCH_3$ | $-CH_2-CH_2-CN$ |
| 3.27 | $2-Cl-3-Cl-C_6H_3-$ | CN | $-CH_2-CH_2-CN$ |
| 3.28 | $2-Cl-3-Cl-C_6H_3-$ | $CF_3$ | $-CH_2-CH_2-CN$ |

Tabelle 3 (Fortsetzung)

| Verb.Nr. | E | $R^1$ | $R^2$ |
|---|---|---|---|
| 3.29 | 2-Cl-3-Cl-$C_6H_3$- | $COOCH_3$ | -$CH_2$-$CH_2$-CN |
| 3.30 | 2-Cl-3-Cl-$C_6H_3$- | $COOC_2H_5$ | -$CH_2$-$CH_2$-CN |
| 3.31 | 2-Cl-3-Cl-$C_6H_3$- | $COOC_3$-$H_7$- | -$CH_2$-$CH_2$-CN |
| 3.32 | 2-Cl-3-Cl-$C_6H_3$- | $COOC_3H_7$-n | -$CH_2$-$CH_2$-CN |
| 3.33 | 2-Cl-3-Cl-$C_6H_3$- | $COOC_4H_9$-n | -$CH_2$-$CH_2$-CN |
| 3.34 | 2-Cl-3-Cl-$C_6H_3$- | $COOC_4H_9$-s | -$CH_2$-$CH_2$-CN |
| 3.35 | 2-Cl-3-Cl-$C_6H_3$- | $COOC_4H_9$-i | -$CH_2$-$CH_2$-CN |
| 3.36 | 2-Cl-4-$CF_3$-$C_6H_3$ | $CF_3$ | -$CH_2$-$CH_2$-CN |
| 3.37 | 2-Cl-4-$CF_3$-$C_6H_3$ | CN | -$CH_2$-$CH_2$-CN |
| 3.38 | 2-Cl-4-$CF_3$-$C_6H_3$ | $COOCH_3$ | -$CH_2$-$CH_2$-CN |
| 3.39 | 4-$CH_3$-$C_6H_4$- | $CF_3$ | -$CH_2$-$CH_2$-CN |
| 3.40 | 4-$CH_3$-$C_6H_4$- | CN | -$CH_2$-$CH_2$-CN |
| 3.41 | 4-$CH_3$-$C_6H_4$- | $COOCH_3$ | -$CH_2$-$CH_2$-CN |
| 3.42 | 2-$CF_3$-4-Cl-$C_6H_3$- | CN | -$CH_2$-$CH_2$-CN |
| 3.43 | 2-$CF_3$-4-Cl-$C_6H_3$- | $CF_3$ | -$CH_2$-$CH_2$-CN |
| 3.44 | 2-$CF_3$-4-Cl-$C_6H_3$- | $COOCH_3$ | -$CH_2$-$CH_2$-CN |
| 3.45 | 2-Cl-3-Cl-$C_6H_3$ | CN | -$CH_2$-$CH_2$-$COOCH_3$ |
| 3.46 | 2-Cl-3-Cl-$C_6H_3$- | $CF_3$ | -$CH_2$-$CH_2$-$COOCH_3$ |
| 3.47 | 2-Cl-3-Cl-$C_6H_3$- | $COOCH_3$ | -$CH_2$-$CH_2$-$COOCH_3$ |
| 3.48 | 2-Cl-3-Cl-$C_6H_3$- | $COOC_2H_5$ | -$CH_2$-$CH_2$-$COOCH_3$ |
| 3.49 | 2-Cl-3-Cl-$C_6H_3$- | $COOC_3H_7$-i | -$CH_2$-$CH_2$-$COOCH_3$ |
| 3.50 | 2-Cl-3-Cl-$C_6H_3$- | $COOC_3H_7$-n | -$CH_2$-$CH_2$-$COOCH_3$ |
| 3.51 | 2-Cl-3-Cl-$C_6H_3$- | $COOC_4H_9$-n | -$CH_2$-$CH_2$-$COOCH_3$ |
| 3.52 | 2-Cl-3-Cl-$C_6H_3$- | $COOC_4H_9$-s | -$CH_2$-$CH_2$-$COOCH_3$ |
| 3.53 | 2-Cl-3-Cl-$C_6H_3$- | $COOC_4H_9$-i | -$CH_2$-$CH_2$-$COOCH_3$ |
| 3.54 | 2-Cl-4-$CF_3$-$C_6H_3$- | $CF_3$ | -$CH_2$-$CH_2$-$COOCH_3$ |
| 3.55 | 2-Cl-4-$CF_3$-$C_6H_3$- | CN | -$CH_2$-$CH_2$-$COOCH_3$ |
| 3.56 | 2-Cl-4-$CF_3$-$C_6H_3$- | $COOCH_3$ | -$CH_2$-$CH_2$-$COOCH_3$ |
| 3.57 | 4-$CH_3$-$C_6H_4$- | $CF_3$ | -$CH_2$-$CH_2$-$COOCH_3$ |
| 3.58 | 4-$CH_3$-$C_6H_4$- | CN | -$CH_2$-$CH_2$-$COOCH_3$ |
| 3.59 | 4-$CH_3$-$C_6H_4$- | $COOCH_3$ | -$CH_2$-$CH_2$-$COOCH_3$ |
| 3.60 | 2-$CF_3$-4-Cl-$C_6H_3$- | CN | -$CH_2$-$CH_2$-$COOCH_3$ |

Tabelle 3 (Fortsetzung)

| Verb.Nr. | E | $R^1$ | $R^2$ |
|---|---|---|---|
| 3.61 | $2-CF_3-4-Cl-C_6H_3-$ | $CF_3$ | $-CH_2-CH_2-COOCH_3$ |
| 3.62 | $2-CF-4-Cl-C_6H_3-$ | $COOCH_3$ | $-CH_2-CH_2-COOCH_3$ |
| 3.63 | $C_6H_5-$ | $CF_3$ | $-CH_2-CH_2-COOCH_3$ |
| 3.64 | $C_6H_5-$ | $COOCH_3$ | $-CH_2-CH_2-COOCH_3$ |
| 3.65 | $C_6H_5-$ | $COOC_2H_5$ | $-CH_2-CH_2-COOCH_3$ |
| 3.66 | $C_6H_5-$ | CN | $-CH_2-CH_2-COOCH_3$ |
| 3.67 | $2-Cl-C_6H_4-$ | $CF_3$ | $-CH_2-CH_2-COOCH_3$ |
| 3.68 | $2-Cl-C_6H_4-$ | $COOC_2H_5$ | $-CH_2-CH_2-COOCH_3$ |
| 3.69 | $2-Cl-C_6H_4-$ | CN | $-CH_2-CH_2-COOCH_3$ |
| 3.70 | $2-Cl-C_6H_4-$ | $COOCH_3$ | $-CH_2-CH_2-COOCH_3$ |
| 3.71 | $2-Cl-C_6H_4-$ | $COOC_3H_7-i$ | $-CH_2-CH_2-COOCH_3$ |
| 3.72 | $3-Cl-C_6H_4-$ | $CF_3$ | $-CH_2-CH_2-COOCH_3$ |
| 3.73 | $3-Cl-C_6H_4-$ | CN | $-CH_2-CH_2-COOCH_3$ |
| 3.74 | $3-Cl-C_6H_4-$ | $COOC_2H_5$ | $-CH_2-CH_2-COOCH_3$ |
| 3.75 | $3-Cl-C_6H_4-$ | $COOC_4H_9-n$ | $-CH_2-CH_2-COOCH_3$ |
| 3.76 | $2-F-C_6H_4-$ | $CF_3$ | $-CH_2-CH_2-COOCH_3$ |
| 3.77 | $2-F-C_6H_4-$ | CN | $-CH_2-CH_2-COOCH_3$ |
| 3.78 | $2-F-C_6H_4-$ | $COOCH_3$ | $-CH_2-CH_2-COOCH_3$ |
| 3.79 | $2-CF_3-C_6H_4-$ | $COOC_2H_5$ | $-CH_2-CH_2-COOCH_3$ |
| 3.80 | $2-CF_3-C_6H_4-$ | $COOCH_3$ | $-CH_2-CH_2-COOCH_3$ |
| 3.81 | $2-CF_3-C_6H_4-$ | $CF_3$ | $-CH_2-CH_2-COOCH_3$ |
| 3.82 | $2-CF_3-C_6H_4-$ | CN | $-CH_2-CH_2-COOCH_3$ |
| 3.83 | $4-Cl-C_6H_4-$ | $COOCH_3$ | $-CH_2-CH_2-COOCH_3$ |
| 3.84 | $4-Cl-C_6H_4-$ | $CF_3$ | $-CH_2-CH_2-COOCH_3$ |
| 3.85 | $4-Cl-C_6H_4$ | CN | $-CH_2-CH_2-COOCH_3$ |
| 3.86 | $2-Cl-4-Cl-C_6H_3-$ | $CF_3$ | $-CH_2-CH_2-COOCH_3$ |
| 3.87 | $2-Cl-4-Cl-C_6H_3-$ | CN | $-CH_2-CH_2-COOCH_3$ |
| 3.88 | $2-Cl-4-Cl-C_6H_3-$ | $COOCH_3$ | $-CH_2-CH_2-COOCH_3$ |
| 3.89 | $2-Br-C_6H_4-$ | | $-CH_2-CH_2COOCH_3$ |
| 3.90 | $C_6H_5-$ | $CF_3$ | $-CH_2-CH_2-COOC_2H_5$ |
| 3.91 | $C_6H_5-$ | $COOCH_3$ | $-CH_2-CH_2-COOC_2H_5$ |
| 3.92 | $C_6H_5-$ | $COOC_2H_5$ | $-CH_2-CH_2-COOC_2H_5$ |

Tabelle 3 (Fortsetzung)

| Verb.Nr. | E | $R^1$ | $R^2$ |
|---|---|---|---|
| 3.93 | $C_6H_5-$ | CN | $-CH_2-CH_2-COOC_2H_5$ |
| 3.94 | $2-Cl-C_6H_4-$ | $CF_3$ | $-CH_2-CH_2-COOC_2H_5$ |
| 3.95 | $2-Cl-C_6H_4-$ | $COOC_2H_5$ | $-CH_2-CH_2-COOC_2H_5$ |
| 3.96 | $2-Cl-C_6H_4-$ | CN | $-CH_2-CH_2-COOC_2H_5$ |
| 3.97 | $2-Cl-C_6H_4-$ | $COOCH_3$ | $-CH_2-CH_2-COOC_2H_5$ |
| 3.98 | $2-Cl-C_6H_4-$ | $COOC_3H_7-i$ | $-CH_2-CH_2-COOC_2H_5$ |
| 3.99 | $3-Cl-C_6H_4-$ | $CF_3$ | $-CH_2-CH_2-COOC_2H_5$ |
| 3.100 | $3-Cl-C_6H_4-$ | CN | $-CH_2-CH_2-COOC_2H_5$ |
| 3.101 | $3-Cl-C_6H_4-$ | $COOC_2H_5$ | $-CH_2-CH_2-COOC_2H_5$ |
| 3.102 | $3-Cl-C_6H_4-$ | $COOC_4H_9-n$ | $-CH_2-CH_2-COOC_2H_5$ |
| 3.103 | $2-F-C_6H_4-$ | $CF_3$ | $-CH_2-CH_2-COOC_2H_5$ |
| 3.104 | $2-F-C_6H_4-$ | CN | $-CH_2-CH_2-COO_2H_5$ |
| 3.105 | $2-F-C_6H_4-$ | $COOCH_3$ | $-CH_2-CH_2-COOC_2H_5$ |
| 3.106 | $2-CF_3-C_6H_4-$ | $COOC_2H_5$ | $-CH_2-CH_2-COOC_2H_5$ |
| 3.107 | $2-CF_3-C_6H_4-$ | $COOCH_3$ | $-CH_2-CH_2-COOC_2H_5$ |
| 3.108 | $2-CF_3-C_6H_4-$ | $CF_3$ | $-CH_2-CH_2-COOC_2H_5$ |
| 3.109 | $2-CF_3-C_6H_4-$ | CN | $-CH_2-CH_2-COOC_2H_5$ |
| 3.110 | $4-Cl-C_6H_4-$ | $COOCH_3$ | $-CH_2-CH_2-COOC_2H_5$ |
| 3.111 | $4-Cl-C_6H_4-$ | $CF_3$ | $-CH_2-CH_2-COOC_2H_5$ |
| 3.112 | $4-Cl-C_6H_4-$ | CN | $-CH_2-CH_2-COOC_2H_5$ |
| 3.113 | $2-Cl-4-Cl-C_6H_3$ | $CF_3$ | $-CH_2-CH_2-COOC_2H_5$ |
| 3.114 | $2-Cl-4-Cl-C_6H_3-$ | CN | $-CH_2-CH_2-COOC_2H_5$ |
| 3.115 | $2-Cl-4-Cl-C_6H_3-$ | $COOCH_3$ | $-CH_2-CH_2-COOC_2H_5$ |
| 3.116 | $2-Cl-3-Cl-C_6H_3-$ | CN | $-CH_2-CH_2-COOC_2H_5$ |
| 3.117 | $2-Cl-3-Cl-C_6H_3-$ | $CF_3$ | $-CH_2-CH_2-COOC_2H_5$ |
| 3.118 | $2-Cl-3-Cl-C_6H_3-$ | $COOCH_3$ | $-CH_2-CH_2-COOC_2H_5$ |
| 3.119 | $2-Cl-3-Cl-C_6H_3-$ | $COOC_2H_5$ | $-CH_2-CH_2-COOC_2H_5$ |
| 3.120 | $2-Cl-3-Cl-C_6H_3-$ | $COOC_3H_7-i$ | $-CH_2-CH_2-COOC_2H_5$ |
| 3.121 | $2-Cl-3-Cl-C_6H_3-$ | $COOC_3H_7-n$ | $-CH_2-CH_2-COOC_2H_5$ |
| 3.122 | $2-Cl-3-Cl-C_6H_3-$ | $COOC_4H_9-n$ | $-CH_2-CH_2-COOC_2H_5$ |
| 3.123 | $2-Cl-3-Cl-C_6H_3-$ | $COOC_4H_9-s$ | $-CH_2-CH_2-COOC_2H_5$ |
| 3.124 | $2-Cl-3-Cl-C_6H_3-$ | $COOC_4H_9-i$ | $-CH_2-CH_2-COOC_2H_5$ |
| 3.125 | $2-Cl-4-CF_3-C_6H_3-$ | $CF_3$ | $-CH_2-CH_2-COOC_2H_5$ |
| 3.126 | $2-Cl-4-CF_3-C_6H_3-$ | CN | $-CH_2-CH_2-COOC_2H_5$ |

Tabelle 3 (Fortsetzung)

| Verb.Nr. | E | $R^1$ | $R^2$ |
|---|---|---|---|
| 3.127 | 2-Cl-4-$CF_3$-$C_6H_3$- | $COOCH_3$ | -$CH_2$-$CH_2$-$COOC_2H_5$ |
| 3.128 | 4-$CH_3$-$C_6H_4$- | $CF_3$ | -$CH_2$-$CH_2$-$COOC_2H_5$ |
| 3.129 | 4-$CH_3$-$C_6H_4$- | CN | -$CH_2$-$CH_2$-$COOC_2H_5$ |
| 3.130 | 4-$CH_3$-$C_6H_4$- | $COOCH_3$ | -$CH_2$-$CH_2$-$COOC_2H_5$ |
| 3.131 | 2-$CF_3$-4-Cl-$C_6H_3$- | CN | -$CH_2$-$CH_2$-$COOC_2H_5$ |
| 3.132 | 2-$CF_3$-4-Cl-$C_6H_3$- | $CF_3$ | -$CH_2$-$CH_2$-$COOC_2H_5$ |
| 3.133 | 2-$CF_3$-4-Cl-$C_6H_3$- | $COOCH_3$ | -$CH_2$-$CH_2$-$COOC_2H_5$ |
| 3.134 | 2-Cl-3-Cl-$C_6H_3$- | CN | -$CH_2$-$CH_2$-$COOC_3H_7$-n |
| 3.135 | 2-Cl-3-Cl-$C_6H_3$- | $CF_3$ | -$CH_2$-$CH_2$-$COOC_3H_7$-n |
| 3.136 | 2-Cl-3-Cl-$C_6H_3$- | $COOCH_3$ | -$CH_2$-$CH_2$-$COOC_3H_7$-n |
| 3.137 | 2-Cl-3-Cl-$C_6H_3$- | $COOC_2H_5$ | -$CH_2$-$CH_2$-$COOC_3H_7$-n |
| 3.138 | 2-Cl-3-Cl-$C_6H_3$- | $COOC_3H_7$-i | -$CH_2$-$CH_2$-$COOC_3H_7$-n |
| 3.139 | 2-Cl-3-Cl-$C_6H_3$- | $COOC_3H_7$-n | -$CH_2$-$CH_2$-$COOC_3H_7$-n |
| 3.140 | 2-Cl-3-Cl-$C_6H_3$- | $COOC_4H_9$-n | -$CH_2$-$CH_2$-$COOC_3H_7$-n |
| 3.141 | 2-Cl-3-Cl-$C_6H_3$- | $COOC_4H_9$-s | -$CH_2$-$CH_2$-$COOC_3H_7$-n |
| 3.142 | 2-Cl-3-Cl-$C_6H_3$- | $COOC_4H_9$-i | -$CH_2$-$CH_2$-$COOC_3H_7$-n |
| 3.143 | 2-Cl-4-$CF_3$-$C_6H_3$- | $CF_3$ | -$CH_2$-$CH_2$-$COOC_3H_7$-n |
| 3.144 | 2-Cl-4-$CF_3$-$C_6H_3$- | CN | -$CH_2$-$CH_2$-$COOC_3H_7$-n |
| 3.145 | 2-Cl-4-$CF_3$-$C_6H_3$- | $COOCH_3$ | -$CH_2$-$CH_2$-$COOC_3H_7$-n |
| 3.146 | 4-$CH_3$-$C_6H_4$- | $CF_3$ | -$CH_2$-$CH_2$-$COOC_3H_7$-n |
| 3.147 | 4-$CH_3$-$C_6H_4$- | CN | -$CH_2$-$CH_2$-$COOC_3H_7$-n |
| 3.148 | 4-$CH_3$-$C_6H_4$- | $COOCH_3$ | -$CH_2$-$CH_2$-$COOC_3H_7$-n |
| 3.149 | 2-$CF_3$-4-Cl-$C_6H_3$- | CN | -$CH_2$-$CH_2$-$COOC_3H_7$-n |
| 3.150 | 2-$CF_3$-4-Cl-$C_6H_3$- | $CF_3$ | -$CH_2$-$CH_2$-$COOC_3H_7$-n |
| 3.151 | 2-$CF_3$-4-Cl-$C_6H_3$- | $COOCH_3$ | -$CH_2$-$CH_2$-$COOC_3H_7$-n |
| 3.152 | $C_6H_5$- | $CF_3$ | -$CH_2$-$CH_2$-$COOC_3H_7$-n |
| 3.153 | $C_6H_5$- | $COOCH_3$ | -$CH_2$-$CH_2$-$COOC_3H_7$-n |
| 3.154 | $C_6H_5$- | $COOC_2H_5$ | -$CH_2$-$CH_2$-$COOC_3H_7$-n |
| 3.155 | $C_6H_5$- | CN | -$CH_2$-$CH_2$-$COOC_3H_7$-n |
| 3.156 | 2-Cl-$C_6H_4$- | $CF_3$ | -$CH_2$-$CH_2$-$COOC_3H_7$-n |
| 3.157 | 2-Cl-$C_6H_4$- | $COOC_2H_5$ | -$CH_2$-$CH_2$-$COOC_3H_7$-n |
| 3.158 | 2-Cl-$C_6H_4$- | CN | -$CH_2$-$CH_2$-$COOC_3H_7$-n |
| 3.159 | 2-Cl-$C_6H_4$ | $COOCH_3$ | -$CH_2$-$CH_2$-$COOC_3H_7$-n |
| 3.160 | 2-Cl-$C_6H_4$- | $COOC_3H_7$-i | -$CH_2$-$CH_2$-$COOC_3H_7$-n |

Tabelle 3 (Fortsetzung)

| Verb.Nr. | E | $R^1$ | $R^2$ |
|---|---|---|---|
| 3.161 | 3-Cl-$C_6H_4$- | $CF_3$ | $-CH_2-CH_2-COOC_3H_7$-n |
| 3.162 | 3-Cl-$C_6H_4$- | CN | $-CH_2-CH_2-COOC_3H_7$-n |
| 3.163 | 3-Cl-$C_6H_4$- | $COOC_2H_5$ | $-CH_2-CH_2-COOC_3H_7$-n |
| 3.164 | 3-Cl-$C_6H_4$- | $COOC_4H_9$-n | $-CH_2-CH_2-COOC_3H_7$-n |
| 3.165 | 2-F-$C_6H_4$- | $CF_3$ | $-CH_2-CH_2-COOC_3H_7$-n |
| 3.166 | 2-F-$C_6H_4$- | CN | $-CH_2-CH_2-COOC_3H_7$-n |
| 3.167 | 2-F-$C_6H_4$- | $COOCH_3$ | $-CH_2-CH_2-COOC_3H_7$-n |
| 3.168 | 2-$CF_3$-$C_6H_4$- | $COOC_2H_5$ | $-CH_2-CH_2-COOC_3H_7$-n |
| 3.169 | 2-$CF_3$-$C_6H_4$- | $COOCH_3$ | $-CH_2-CH_2-COOC_3H_7$-n |
| 3.170 | 2-$CF_3$-$C_6H_4$- | $CF_3$ | $-CH_2-CH_2-COOC_3H_7$-n |
| 3.171 | 2-$CF_3$-$C_6H_4$- | CN | $-CH_2-CH_2-COOC_3H_7$-n |
| 3.172 | 4-Cl-$C_6H_4$- | $COOCH_3$ | $-CH_2-CH_2-COOC_3H_7$-n |
| 3.173 | 4-Cl-$C_6H_4$- | $CF_3$ | $-CH_2-CH_2-COOC_3H_7$-n |
| 3.174 | 4-Cl-$C_6H_4$- | CN | $-CH_2-CH_2-COOC_3H_7$-n |
| 3.175 | 2-Cl-4-Cl-$C_6H_3$- | $CF_3$ | $-CH_2-CH_2-COOC_3H_7$-n |
| 3.176 | 2-Cl-4-Cl-$C_6H_5$- | CN | $-CH_2-CH_2-COOC_3H_7$-n |
| 3.177 | 2-Cl-4-Cl-$C_6H_3$- | $COOCH_3$ | $-CH_2-CH_2-COOC_3H_7$-n |
| 3.178 | 2-Cl-3-Cl-$C_6H_3$ | CN | $-CH{=}CH_2$ |
| 3.179 | 2-Cl-3-Cl-$C_6H_3$ | $CF_3$ | $-CH{=}CH_2$ |
| 3.180 | 2-Cl-3-Cl-$C_6H_3$ | CN | $-CH_2CH_2-Cl$ |
| 3.181 | 2-Cl-3-Cl-$C_6H_3$ | $CF_3$ | $-CH_2-CH_2-Cl$ |
| 3.182 | 2-Cl-3-Cl-$C_6H_3$ | $CF_3$ | $-CH_2-CH_2-SO_2-$ 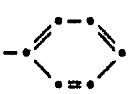 $-CH_3$ |
| 3.183 | 2-Cl-3-Cl-$C_6H_3$ | CN | $-CH_2-CH_2-SO_2-$ 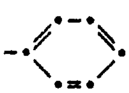 $-CH_3$ |

Tabelle 4

$$E - CH = CCl - CF_3$$

| Verb.Nr. | E |
|---|---|
| 4.1 | $C_6H_5-$ |
| 4.2 | $2-Cl-C_6H_4-$ |
| 4.3 | $3-Cl-C_6H_4-$ |
| 4.4 | $4-Cl-C_6H_4-$ |
| 4.5 | $2-F-C_6H_4-$ |
| 4.6 | $2-Br-C_6H_4-$ |
| 4.7 | $2-CF3-C_6H_4-$ |
| 4.8 | $2.CF-4-Cl-C_6H_3-$ |
| 4.9 | $2-Cl-4-Cl-C_6H_3-$ |
| 4.10 | $2-Cl-3-Cl-C_6H_3-$ |
| 4.11 | $2-Cl-4-CF_3-C_6H_3-$ |
| 4.12 | $4-CH_3-C_6H_4-$ |

Tabelle 5

$$E - \underset{OH}{CH} - CCl_2 - CF_3$$

| Verb.Nr. | E |
|---|---|
| 5.1 | $C_6H_5-$ |
| 5.2 | $2-Cl-C_6H_4-$ |
| 5.3 | $3-Cl-C_6H_4-$ |
| 5.4 | $4-Cl-C_6H_4-$ |
| 5.5 | $2-F-C_6H_4-$ |
| 5.6 | $2-Br-C_6H_4-$ |
| 5.7 | $2-CF_3-C_6H_4-$ |
| 5.8 | $2-CF_3-4-Cl-C_6H_3-$ |
| 5.9 | $2-Cl-4-Cl-C_6H_3-$ |
| 5.10 | $2-Cl-3-Cl-C_6H_3-$ |
| 5.11 | $2-Cl-4-CF_3-C_6H_3-$ |
| 5.12 | $4-CH_3-C_6H_4-$ |

Tabelle 6

$$E - \underset{|}{C}H - CCl_2 - CF_3$$
$$O\text{-}COCH_3$$

| Verb.Nr. | E |
|---|---|
| 6.1 | $C_6H_5$- |
| 6.2 | 2-Cl-$C_6H_4$- |
| 6.3 | 3-Cl-$C_6H_4$- |
| 6.4 | 4-Cl-$C_6H_4$- |
| 6.5 | 2-F-$C_6H_4$- |
| 6.6 | 2-Br-$C_6H_4$- |
| 6.7 | 2-$CF_3$-$C_6H_4$- |
| 6.8 | 2-$CF_3$-4-Cl-$C_6H_3$- |
| 6.9 | 2-Cl-4-Cl-$C_6H_3$- |
| 6.10 | 2-Cl-3-Cl-$C_6H_3$- |
| 6.11 | 2-Cl-4-$CF_3$-$C_6H_3$- |
| 6.12 | 4-$CH_3$-$C_6H_4$- |

Formulierungsbeispiele für flüssige Wirkstoffe der Formel I (% = Gewichtsprozent)

| Fl. Emulsion-Konzentrate | a) | b) | c) |
|---|---|---|---|
| Wirkstoff aus der Tabelle 3 | 25 % | 40 % | 50 % |
| Ca-Dodecylbenzolsulfonat | 5 % | 8 % | 6 % |
| Ricinusöl-polyäthylenglykoläther (36 Mol Aethylenoxid | 5 % | - | - |
| Tributylphenol-polyäthylenglykoläther (30 Mol Aethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus der Tabelle 3 | 80 % | 10 % | 5 % | 95 % |
| Aethylenglykol-monomethyläther | 20 % | - | - | - |
| Polyäthylenglykol M G 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

| F3. Granulate | a) | b) |
|---|---|---|
| Wirkstoff aus der Tabelle 3 | 5 % | 10 % |
| Kaolin | 94 % | |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus der Tabelle 3 | 2 % | 5 % |
| Hochdisperse Kieselsäure | 1 % | 5 % |
| Talkum | 97 % | - |
| Kaolin | - | 90 % |

Durch inniges Vermischen auf Trägerstoffe mit dem Wirkstoff erhält man gebrauchsfertige Stäubemittel.

Formulierungsbeispiele für feste Wirkstoffe der Formel I
(% = Gewichtsprozent

| F5. Spritzpulver | a) | b) | c) |
|---|---|---|---|
| Wirkstoff aus der Tabelle 3 | 25 % | 50 % | 75 % |
| Na-Ligninsulfonat | 5 % | 5 % | - |
| Na-Laurylsulfat | 3 % | - | 5 % |

| | | | |
|---|---|---|---|
| Na-Diisobutylnaphthalinsulfonat | - | 6 % | 10 % |
| Octylphenolpolyäthylenglykoläther (7-8 Mol Aethylenoxid | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

F6. Emulsions-Konzentrat

| | |
|---|---|
| Wirkstoff aus der Tabelle 3 | 10 % |
| Octylphenolpolyäthylenglykoläther (4-5 Mol Aethylenoxid) | 3 % |
| Ca-Dodecylbenzolsulfonat | 3 % |
| Ricinusölpolyglykoläther (35 Mol Aethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus der Tabelle 3 | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

F8. Extruder Granulat

| | |
|---|---|
| Wirkstoff aus der Tabelle 3 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wurd extrudiert und anschliessend im Luftstrom getrocknet.

F9. Umhüllungs-Granulat

| | |
|---|---|
| Wirkstoff aus der Tabelle 3 | 3 % |
| Polyäthylenglykol MG 200 | 3 % |
| Kaolin | 94 % |

(MG = Molekulargewicht)

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus der Tabelle 3 | 40 % |
| Aethylenglykol | 10 % |
| Nonylphenolpolyäthyleglykoläther (15 Mol Aethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig veermischt. Man erhält so ein Suspensions-Konzentrat, aus welchem durch Verdünnen mit Wasser Suspensionen jeder gewünschten Konzentration hergestellt werden können.

Biologische Beispiele:

Beispiel B1: Wirkung gegen Botrytis cinerea auf Bohnen
Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21°C erfolgt die Beurteilung des Pilzbefalles. Die Verbindungen aus der Tabelle 3 hemmen nicht nur im obigen Modellversuch sondern auch im Freiland die Pilzinfektion sehr stark. Bei einer Konzentration von 0,02 % erweisen sich z.B. die Verbindungen aus der Tabelle 3 als voll wirksam (Krankheitsbefall 0 bis 5 %). Befall unbehandelter aber infizierter Bohnenpflanzen beträgt 100 %.

Beispiel B2: Wirkung gegen Botrytis cinerea an Apfelfrüchten

Künstlich verletzte Aepfel werden behandelt, indem eine aus Spritzpulver der Wirksubstanz hergestellte Spritzbrühe auf die Veletzungsstellen aufgetropft wird. Die behandelten Früchte werden anschliessend mit einer Sporensuspension von Botrytis cinerea inokuliert und während einer Woche bei einer hohen Luftfeuchtigkeit und ca. 20°C inkubiert.

Bei der Auswertung werden die angefaulten Verletzungsstellen gezählt und daraus die fungizide Wirkung der Testsubstanz abgeleitet. Unter anderem hemmen die Verbindungen aus der Tabelle 3 den Pilzbefall im Vergleich zu unbehandelten Kontrollfrüchten (100 % Befall) fast vollständig.

Patentansprüche

1. 3-Phenylpyrrol-Derivate der Formel I

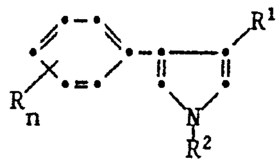

(I) ,

worin $R^1$ für Cyan, Trifluormethyl oder $C_1$-$C_4$-Alkoxycarbonyl, $R^2$ für Vinyl oder eine Gruppe $-CH_2-CH_2-X$, in der X Chlor, Brom, Cyan, $C_1$-$C_4$-Alkoxycarbonyl, Phenylsulfonyl, 4-Bromphenylsulfonyl oder 4-Methylphenylsulfonyl bedeutet, R für Halogen, $C_1$-$C_4$-Alkyl oder $C_1$-$C_4$-Halogenalkyl und n für eine Zahl null, eins oder zwei stehen.

2. Verbindungen gemäss Anspruch 1, dadurch gekennzeichnet, dass die Phenylgruppe in 2- und 3-Stellung durch zwei Chloratome substituiert ist.

3. Verbindungen gemäss Anspruch 1, dadurch gekennzeichnet, dass $R^1$ für Cyan oder Trifluormethyl steht.

4. Verbindungen gemäss Anspruch 1, dadurch gekennzeichnet, dass $R^2$ für Aethoxycarbonyläthyl oder 2-Cyanoäthyl steht.

5. Verbindungen gemäss Anspruch 1, dadurch gekennzeichnet, dass die Phenylgruppe in 2- und/oder 3-Stellung durch Chloratome substituiert ist, $R^1$ für Trifluormethyl oder Cyan und $R^2$ für Aethoxycarbonyläthyl oder 2-Cyanoäthyl stehen.

6. 1-Aethoxycarbonyläthyl-3-(2,3-dichlorphenyl)-4-trifluormethyl-pyrrol gemäss Anspruch 1.

7. 1-Aethoxycarbonyläthyl-4-cyano-3-(2,3-dichlorphenyl)-pyrrol gemäss Anspruch 1.

8. Pflanzenmikrobizides Mittel, dadurch gekennzeichnet, dass es neben Träger- und/oder anderen Zuschlagstoffen als Wirkstoff ein 3-Phenylpyrrol-Derivat der Formel I, gemäss Anspruch 1, enthält.

9. Verfahren zur Herstellung von 3-Phenylpyrrol-Derivaten der Formel I

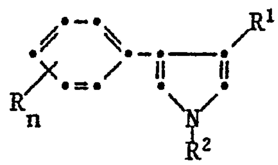

(I) ,

worin $R^1$ für Cyan, Trifluormethyl oder $C_1$-$C_4$-Alkoxycarbonyl, $R^2$ für Vinyl oder eine Gruppe $-CH_2-CH_2-X$, in der X Chlor, Brom, Cyan, $C_1$-$C_4$-Alkoxycarbonyl, Phenylsulfonyl, 4-Bromphenylsulfonyl oder 4-Methylphenylsulfonyl bedeutet, R für Halogen, $C_1$-$C_4$-Alkyl oder $C_1$-$C_4$-Halogenalkyl und n für eine Zahl null, eins oder zwei stehen, dadurch gekennzeichnet, dass man ein Formylglycin-Derivat der Formel II

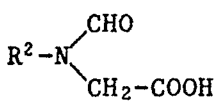

(II) ,

worin $R^2$ die unter Formel I angegebene Bedeutung hat, in Gegenwart eines Kondensationsmittels mit einem Phenylacetylen-Derivat der Formel III

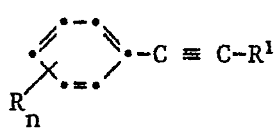

(III) ,

worin $R^1$ und n die unter Formel I gegebenen Bedeutungen haben, behandelt.

10. Verfahren gemäss Anspruch 9, dadurch gekennzeichnet, dass man als Kondensationsmittel Essigsäureanhydrid verwendet.

11. Verfahren gemäss Anspruch 9, dadurch gekennzeichnet, dass man das Verfahren in einem inerten organischen Lösungsmittel bei einer Temperatur zwischen 90° und 150°C durchführt.

12. Formylglycin-Derivate der Formel II

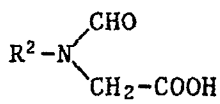

(II) ,

worin $R^2$ die unter Formel I in Anspruch 1 gegebene Bedeutung hat.

13. Verfahren zur Herstellung der Verbindungen der Formel I gemäss Anspruch 1, worin $R^1$ für Trifluormethyl steht, dadurch gekennzeichnet, dass man aus einem Styrolderivat der Formel IV

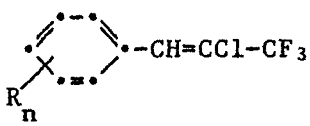

(IV) ,

worin R und n die unter Formel I gegebene Bedeutung haben, in Gegenwart einer Base Chlorwasserstoff abspaltet und das entstandene Trifluormethyl-phenylacetylen-Derivat der Formel IIIa

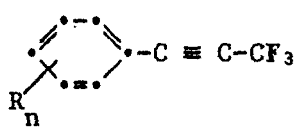

(IIIa) ,

worin R und n die unter Formel I gegebene Bedeutung haben, in Gegenwart eines Kondensationsmittels mit einem Formylglycin-Derivat der Formel II

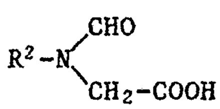

(II) ,

worin $R^2$ die unter Formel I gegebene Bedeutung hat, umsetzt.

14. Trifluormethyl-phenylacetylen-Derivate der Formel IIIa

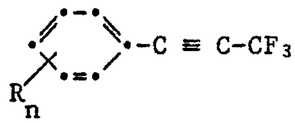

(IIIa) ,

worin R für Halogen, $C_1$-$C_4$-Alkyl oder $C_1$-$C_4$-Halogenalkyl und n für eine Zahl eines oder zwei stehen.

15. Verwendung der 3-Phenylpyrrol-Derivate der Formel I gemäss Anspruch 1 zur Bekämpfung von phytopathogenen Mikroorganismen.

16. Verfahren zur Bekämpfung von phytopathogenen Mikroorganismen, dadurch gekennzeichnet, dass man eine wirksame Menge eines 3-Phenylpyrrol-Derivates der Formel I auf die Pflanze oder deren Lebensraum appliziert.

17. Verfahren gemäss Anspruch 16, dadurch gekennzeichnet, dass es sich bei den Mikroorganismen um phytopathogene Pilze handelt.

18. Verfahren gemäss Anspruch 16, dadurch gekennzeichnet, dass es sich bei den Mikroorganismen um Pilze der Familie Fungi imperfecti, insbesondere Botrytis, handelt.

FO 7.5/CW/bg*